(19) Europäisches Patentamt
European Patent Office
Office européen des brevets

(11) Veröffentlichungsnummer: **0 073 454**
**B1**

(12) # EUROPÄISCHE PATENTSCHRIFT

(45) Veröffentlichungstag der Patentschrift: **02.05.85**

(51) Int. Cl.⁴: **C 07 D 499/68**, A 61 K 31/43

(21) Anmeldenummer: **82107745.0**

(22) Anmeldetag: **24.08.82**

(54) **Neue 6alpha-Methoxy-penicilline, ihre Salze, Verfahren zu ihrer Herstellung und diese Verbindungen enthaltende Arzneimittel.**

(30) Priorität: **02.09.81 DE 3134776**

(43) Veröffentlichungstag der Anmeldung: **09.03.83 Patentblatt 83/10**

(45) Bekanntmachung des Hinweises auf die Patenterteilung: **02.05.85 Patentblatt 85/18**

(84) Benannte Vertragsstaaten: **AT BE CH DE FR IT LI LU NL SE**

(56) Entgegenhaltungen:
**EP - A - 0 003 814**
**EP - A - 0 091 755**
**DE - A - 2 710 979**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Wetzel, Bernd, Dr., Dipl.-Chem., Kapellenweg 21, D-7950 Biberach 1 (DE)**
Erfinder: **Eberlein, Wolfgang, Dr., Dipl.-Chem., Obere Au 6, D-7950 Biberach 1 (DE)**
Erfinder: **Trummlitz, Günter, Dr., Dipl.-Chem., Buchenweg 27, D-7951 Warthausen (DE)**
Erfinder: **Woitun, Eberhard, Dr., Dipl.-Chem., Stecherweg 17, D-7950 Biberach 1 (DE)**
Erfinder: **Maier, Roland, Dr., Dipl.-Chem., Bodelschwinghstrasse 39, D-7950 Biberach 1 (DE)**
Erfinder: **Reuter, Wolfgang, Dr., Dipl.-Chem., Nelkenweg 10, D-7951 Laupertshausen (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue 6 α-Methoxy-penicilline der allgemeinen Formel I

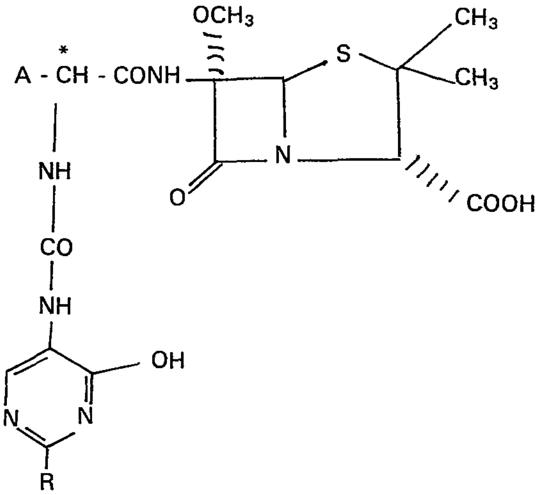
,(I)

ihre physiologisch verträglichen Salze mit anorganischen oder organischen Basen, Verfahren zur Herstellung dieser Verbindungen und diese Verbindungen enthaltende Arzneimittel.

In der obigen allgemeinen Formel I bedeutet:

A die Phenyl-, p-Hydroxyphenyl-, 2- oder 3-Thienylgruppe,

R die Cyclopropylgruppe, eine Gruppe der allgemeinen Formel

$$- NH - R_1, \qquad \text{wobei}$$

$R_1$ einen gegebenenfalls ab Kohlenstoffatom 2 durch eine Hydroxygruppe substituierten aliphatischen, verzweigten oder unverzweigten Kohlenwasserstoffrest mit 1 bis 4 (eventuell ausser 1 und 2) Kohlenstoffatomen, einen gegebenenfalls durch eine Hydroxygruppe substituierten Cycloalkylrest mit 3 bis 6 Kohlenstoffatomen oder eine gegebenenfalls durch eine Hydroxy-, Methylsulfinyl-, Methylsulfonyl- oder Aminosulfonylgruppe substituierte 3-Pyridyl-, 2-Furylmethyl-, 2-Thienylmethyl-, 3-Imidazolylmethyl-, 2-Thiazolylmethyl- oder 3-Pyridylmethylgruppe darstellt,

eine Gruppe der allgemeinen Formel

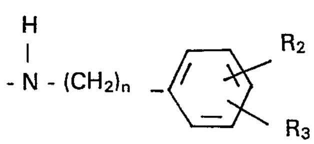
, wobei

n die Zahl 0 oder 1,

$R_2$ und $R_3$, die gleich oder verschieden sein können Wasserstoffatome, Hydroxy-, Acetylamino-, Aminocarbonylamino-, Nitro-, Aminocarbonyl-, Cyan-, Methylsulfinyl-, Methylsulfonyl-, Aminosulfonyl-, Methylaminosulfonyl-, Aminocarbonylmethylenaminosulfonyl-, 2'-Hydroxyäthylaminosulfonyl-, Cyanaminosulfonyl-, Aminocarbonylaminosulfonyl-, Acetylaminosulfonyl-, Methylsulfonylaminosulfonyl- oder Acetylhydrazinosulfonylgruppen darstellen,

eine Gruppe der allgemeinen Formel

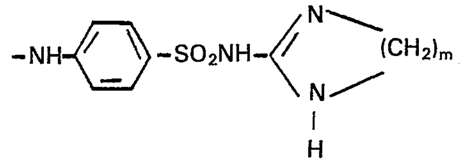
, wobei

m die Zahl 2, 3 oder 4 darstellt, oder

eine Gruppe der Formel

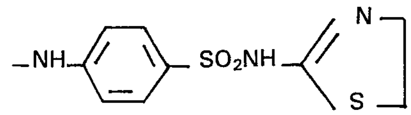

Der Rest R hat vorzugsweise folgende Bedeutungen:

die p-Aminosulfonylanilino-, p-Methylsulfinylanilino-, p-Methylsulfonylanilino-, m-Hydroxy-p-aminosulfonylanilino-, p-Aminocarbonylmethylenamino-sulfonylanilino-, p-(4',5'-Dihydro-imidazol-2'-yl)-aminosulfonylanilino-, p-(3',4',5',6'-Tetrahydro-pyrimidin-2'-yl)-aminosulfonylanilino-, p-(4',5',6',7'-Tetrahydro-1,3-diazepin-2'-yl)-aminosulfonylanilino-, p-(4',5'-Dihydro-thiazol-2'-yl)-aminosulfonylanilino-, p-Hydroxybenzylamino-, p-Aminosulfonylbenzylamino-, Isopropylamino-, 4'-Hydroxycyclohexylamino-, 5'-Aminosulfonyl-2'-thienylmethylamino-, 2'-Furylmethylamino-, 3'-Pyridylmethylamino- oder 4'-Hydroxy-3'-pyridylaminogruppe.

Die Penicillinverbindungen der allgemeinen Formel I können in zwei tautomeren Formen (nämlich vom Lactim- und vom Lactamtyp) vorliegen. Es hängt besonders vom jeweiligen Lösemittel und von der Art des Substituenten R ab, welche der beiden Formen I oder I' überwiegt:

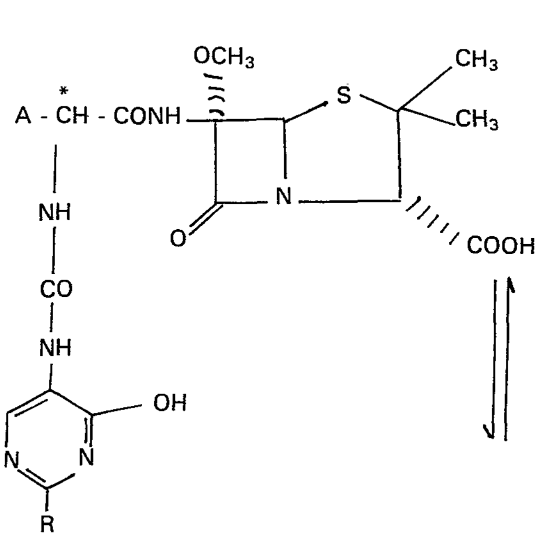
,(I)

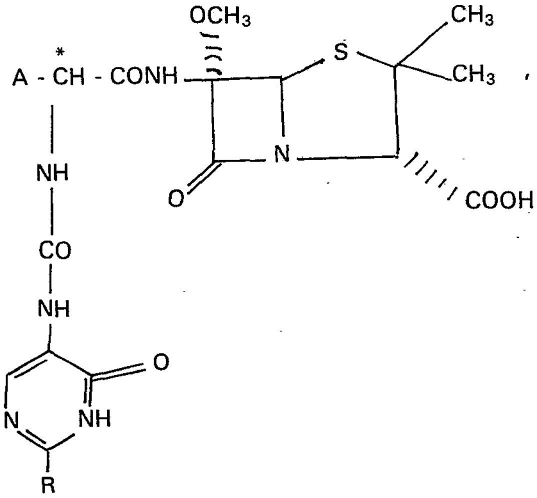

, (I')

Es versteht sich von selbst, dass die eingangs angegebenen Verbindungen vom Typ I immer beide Tautomeren umfassen.

Die Verbindungen der allgemeinen Formel I können bezüglich des Chiralitätszentrums C+ in den beiden möglichen R- und S-Konfigurationen, jedoch auch als ein Gemisch dieser beiden Konfigurationen vorliegen. Besonders bevorzugt sind solche Verbindungen, für welche die D = R-Konfiguration zutrifft.

Die Verbindungen der allgemeinen Formel I lassen sich wie folgt darstellen:

Durch Umsetzung einer Verbindung der allgemeinen Formel II,

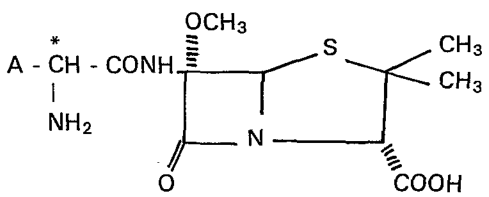

, (II)

in der A wie oben definiert ist, mit einem gegebenenfalls im Reaktionsgemisch hergestellten Pyrimidinderivat der allgemeinen Formel III

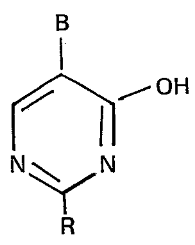

, (III)

oder

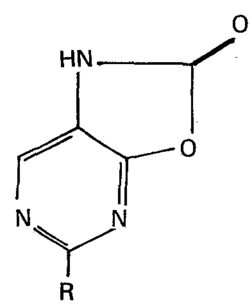

, (IIIa)

in denen

R wie oben definiert ist und

B die Gruppe -NCO oder ein reaktives Derivat der Gruppe NHCOOH bedeutet, wie z.B. die Gruppen -NHCOCl, -NHCOBr oder -NH-COO- 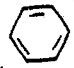 $-NO_2$, wobei die Gruppe NHCOCl besonders bevorzugt ist. Es können auch Gemische von solchen Pyrimidinderivaten der allgemeinen Formel III verwendet werden, in der B teils die eine und teils die andere der vorstehend genannten Bedeutungen besitzt, z.B. die Gruppen -NCO und $-\underset{H}{\underset{|}{N}}COCl$ gleichzeitig nebeneinander.

Die Ausgangsprodukte der allgemeinen Formel II können in Form ihrer anorganischen oder organischen Salze, z.B. als Triäthylammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Dimethylsulfoxid oder Alkoholen, z.B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z.B. halogenierten Kohlenwasserstoffen, wie Chloroform oder Methylenchlorid unter Zusatz von Basen, vorzugsweise Triäthylamin, Diäthylamin oder N-Äthylpiperidin, durchzuführen. Weiterhin lässt sich die Reaktion in einem Gemenge aus Wasser und einem mit Wasser nicht mischbaren Lösungsmittel, wie Äther, z.B. Diäthyläther, halogenierten Kohlenwasserstoffen, z.B. Chloroform oder Methylenchlorid, Schwefelkohlenstoff, Ketonen, z.B. Isobutylmethylketon, Estern, z.B. Essigsäureäthylester, aromatischen Lösungsmitteln, z.B. Benzol, ausführen, wobei es zweckmässig ist, kräftig zu rühren und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Verwendet man als Ausgangsprodukte für das erfindungsgemässe Verfahren die Silylderivate der Verbindungen der allgemeinen Formel II (z.B. Mono- oder Di-trimethylsilylderivate) und setzt sie mit Verbindungen der allgemeinen Formel III bzw. IIIa um, so arbeitet man im allgemeinen zweckmässigerweise in wasser- und hydroxyl-gruppenfreien Lösungsmitteln, beispielsweise in halogenierten Kohlenwasserstoffen, z.B. Methylenchlorid, Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmässigerweise tertiäre aliphatische oder aromatische Amine, wie Pyridin oder Triäthylamin,

oder durch sterische Hinderung schweracylierbare sekundäre Amine, wie Dicyclohexylamin, benützt.

Statt der Silylester können auch alle anderen Carboxylderivate der 6 α-Methoxypenicillinderivate der allgemeinen Formel II, die auf dem Gebiet der Herstellung halbsynthetischer Penicilline bekannt sind, verwendet werden. Typische Beispiele sind die Tritylester, die p-Nitrobenzylester, die Phenacylester oder die β,β,β-Trichloräthylester. Im Anschluss an die Umsetzungen können diese Derivate nach bekannten Methoden in die erfindungsgemässen Penicilline umgewandelt werden. Die Menge der verwendeten Basen ist, z.B. durch die gewünschte Einhaltung eines bestimmten pH-Wertes, festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen oder anorganischen Basen verwendet werden, wie Alkali- und Erdalkalihydroxide, Erdalkalioxide, Alkali- und Erdalkalicarbonate und -hydrogencarbonate, Ammoniak, primäre, sekundäre und tertiäre aliphatische und aromatische Amine sowie heterocyclische Basen. Beispielhaft seien Natrium-, Kalium- und Calziumhydroxid, Calziumoxid, Natrium- und Kaliumcarbonat, Natrium- und Kaliumhydrogencarbonat, Äthylamin, Methyl-äthylamin, Triäthylamin, Hydroxyäthylamin, Anilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z.B. Phosphatpuffer, Citratpuffer und Tris(hydroxy-methyl)amino-methan-Puffer.

Die Reaktionstemperaturen können in einem grösseren Bereich variiert werden. Im allgemeinen arbeitet man zwischen etwa —20 und etwa +50°C, vorzugsweise zwischen 0 und +20°C.

Die Reaktionspartner der allgemeinen Formeln II und III bzw. IIIa können von vornherein in äquimolaren Mengen miteinander zur Reaktion gebracht werden. Es kann aber in einzelnen Fällen durchaus zweckmässig sein, einen der beiden Reaktionspartner im Überschuss zu verwenden, um sich damit die Reinigung des Endproduktes zu erleichtern oder um die Ausbeute zu steigern.

Die Aufarbeitung des Reaktionsgemisches nach erfolgter Umsetzung wird nach den bei β-Lactam-Antibiotika gebräuchlichen Methoden vorgenommen; dasselbe gilt für die Isolierung und Reinigung der Endprodukte, beispielsweise für die Freisetzung der Säure aus ihren Salzen und die Überführung der freien Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat oder die Zugabe äquimolekularer Mengen von Kalium- oder Natriumhydrogencarbonat und anschliessende Gefriertrocknung.

Die als Ausgangsstoffe verwendeten 6 α-Methoxy-penicillin-derivate der allgemeinen Formel II sind literaturbekannt oder können in Analogie zu literaturbekannten Methoden dargestellt werden (siehe z.B. Bentley et al. J. chem. Soc. 1979, S. 2455, US-A-4 044 000 oder US-A-4 035 359).

Die Ausgangsstoffe der allgemeinen Formel III bzw. IIIa können beispielsweise durch Umsetzung der entsprechenden 5-Aminopyrimidine der allgemeinen Formel IV

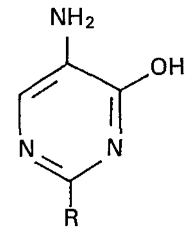

, (IV)

in der R wie oben definiert ist, mit Phosgen gewonnen werden. Diese Umsetzung erfolgt vorzugsweise in einem nicht Hydroxylgruppen-enthaltenden Lösungsmittel, wie Tetrahydrofuran, Methylenchlorid, Chloroform, Dimethoxyäthan oder Hexametapol bei Temperaturen zwischen —40° und +60°C, vorzugsweise zwischen —10° und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuss verwendet werden. Sollten die betreffenden Aminopyrimidine der allgemeinen Formel IV in einem der erwähnten Lösungsmittel schwer löslich sein, kann die Phosgenierung auch in heterogener Phase durchgeführt werden. Desweiteren können die Aminopyrimidine der allgemeinen Formel IV durch Behandlung mit einem Silylierungsmittel, wie Hexamethyldisilazan oder Trimethylchlorsilan/Triäthylamin oder Trimethylsilyldiäthylamin, in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel III bzw. IIIa reagiert. Je nach der Art des Lösungsmittels, der Höhe der Temperatur, der Menge und Art der eingesetzten Base entsteht entweder überwiegend das entsprechende Isocyanat oder Carbaminsäurehalogenid oder ein Gemisch dieser beiden Verbindungen.

Die durch Phosgenierung entstandenen Ausgangsprodukte der allgemeinen Formel III, IIIa bzw. deren Gemische sind in den obenerwähnten Lösungsmitteln im allgemeinen gut löslich und können, nach Entfernung des überschüssigen Phosgens, ohne weitere Reinigung mit den entsprechenden Penicillinderivaten der allgemeinen Formel II direkt umgesetzt werden. Die Synthese der Aminopyrimidine der allgemeinen Formel IV wird im US-A-4 241 056 beschrieben.

In der folgenden Tabelle werden typische, besonders gut wirksame Penicilline gemäss vorliegender Erfindung aufgezählt.

| A | R |
|---|---|
| Phenyl- | p-Sulfamoylanilino- |
| p-Hydroxyphenyl- | p-Sulfamoylanilino- |
| 2-Thienyl- | p-Sulfamoylanilino- |
| 3-Thienyl- | p-Sulfamoylanilino- |
| Phenyl- | m-Hydroxy-p-sulfamoyl-anilino- |
| p-Hydroxyphenyl- | m-Hydroxy-p-sulfamoyl-anilino- |
| p-Hydroxyphenyl- | p-Methylsulfinylanilino- |
| Phenyl- | p-Methylsulfonylanilino- |
| p-Hydroxyphenyl- | p-Methylsulfonylanilino- |
| p-Hydroxyphenyl- | 4′-Hydroxycyclohexyl-amino- |
| p-Hydroxyphenyl- | p-Hydroxybenzylamino- |
| Phenyl- | 5′-Sulfamoyl-2′-thienyl-methylamino- |
| p-Hydroxyphenyl- | 5′-Sulfamoyl-2′-thienyl-methylamino- |
| 2-Thienyl- | 5′-Sulfamoyl-2′-thienyl-methylamino- |
| Phenyl- | 2′-Furylmethylamino- |
| p-Hydroxyphenyl- | 2′-Furylmethylamino- |
| Phenyl- | 3′-Pyridylmethylamino- |
| p-Hydroxyphenyl- | 3′-Pyridylmethylamino- |
| p-Hydroxyphenyl- | 4′-Hydroxy-3′-pyridyl-amino- |
| Phenyl- | 4′-Hydroxy-3′-pyridyl-amino- |
| p-Hydroxyphenyl- | p-Aminocarbonylmethyl--sulfamoylanilino- |
| p-Hydroxyphenyl- | p-(4′,5′-Dihydro-imidazol--2′-yl)-sulfamoylanilino- |
| p-Hydroxyphenyl- | p-(3′,4′,5′,6′-Tetrahydro--pyrimidin-2′-yl)--sulfamoylanilino- |
| p-Hydroxyphenyl- | p-(4′,5′,6′,7′-Tetrahydro--1,3-diazepin-2′-yl)--sulfamoylanilino- |
| p-Hydroxyphenyl- | p-(4′,5′-Dihydro-thiazol--2′-yl)-sulfamoylanilino- |

Während 7 α-Methoxycephalosporin-Derivate in den letzten Jahren eine grosse Bedeutung in der Chemotherapie erlangt haben, ist es bisher kaum gelungen, 6 α-Methoxypenicilline zu synthetisieren, die neben einer hohen β-Lactamase-Stabilität auch über gute antibakterielle Wirkung verfügen.

Solche 6 α-Methoxypenicilline werden z.B. in der EP-A-0 029 871 und EP-A-0 015 690 sowie in der DE-OS 2 732 104 beschrieben. Als Entwicklungssubstanz ist bisher das 6-Methoxy-Derivat des Ticarcillins der Formel

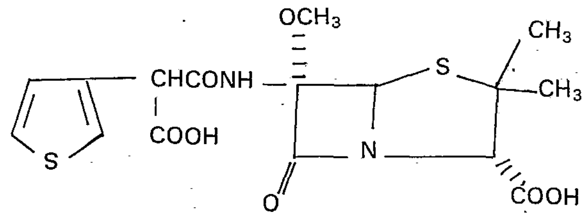

(Temocillin) bekannt geworden (z.B. J. Antimicrobial Agents and Chemoterapy 20, 38-46 [1981]).

Es wurde nun gefunden, dass eine Reihe der durch die allgemeine Formel I repräsentierten Penicillin-Derivate eine ausgezeichnete antibakterielle Wirkung insbesondere gegen gramnegative Bakterien, wie E. coli, Kl. pneumoniae, E. cloacae, Proteus species, Serratia marcescens und auch Pseudomonas-Arten besitzen, die die des Temocillins in vitro und in vivo deutlich übertreffen.

Daneben zeigen sie ausgezeichnete Stabilität gegen verschiedene β-Lactamasen und besitzen damit Wirkungen gegen β-Lactamase-tragende Bakterien.

Ausserdem sind die erfindungsgemässen Wirkstoffe sehr gut verträglich, sie können daher zur Prophylaxe und Chemotherapie von lokalen und systemischen Infektionen in der Human- und Tiermedizin verwendet werden.

Als Krankheiten, die durch die erfindungsgemässen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen.

Dieses wird, wie bereits dargelegt, dadurch ermöglicht, dass die Verbindungen der allgemeinen Formel I sowohl in vitro als auch in vivo gegen schädliche Mikroorganismen, insbesondere gegen grampositive und gramnegative Bakterien und bakterienähnliche Mikroorganismen, sehr stark wirken, wobei sie sich besonders durch ein breites Wirkungsspektrum auszeichnen.

Mit diesen Penicillinderivaten können beispielsweise lokale und/oder systemische Erkrankungen behandelt und/oder verhindert werden, die durch die folgenden Erreger oder durch Mischungen der folgenden Erreger verursacht werden:

Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe;
Klebsiella-Bakterien, z.B. K. pneumoniae;
Proteae-Bakterien der Proteus-Gruppe, z.B. Proteus vulgaris;
Salmonella-Bakterien, z.B. S.thyphimurium;
Shigella-Bakterien, z.B. Shigella dysenteriae;
Pseudomonas-Bakterien, z.B. Pseudomonas aeruginosa;
Aeromonas-Bakterien, z.B. Aeromonas lique faciens;
Spirillaceae, wie Vibrio-Bakterien, z.B. Vibrio cholerae;

Parvobacteriaceae oder Brucellaceae, wie Pasteurella-Bakterien;
Brucella-Bakterien, z.B. Brucella abortus;
Haemophilus-Bakterien, z.B. Haemophilus influenzae;
Bordetella-Bakterien, z.B. Bordetella pertussis;
Moraxella-Bakterien, z.B. Moraxella lacunata;
Bacteroidaceae, wie Bacteroides-Bakterien;
Fusiforme-Bakterien, z.B. Fusobacterium fusiforme;
Sphaerophorus-Bakterien, z.B. Sphaerophorus necrophorus;
Bacillaceae, wie aerobe Sporenbildner, z.B. Bacillus anthracis;
anaerobe Sporenbildner-Chlostridien, z.B. Chlostridium perfringens;
Spirochaetaceae, wie Borrelia-Bakterien;
Treponema-Bakterien, z.B. Treponema pallidum;
Leptospira-Bakterien, wie Leptospira interrogans.

Die obige Aufzählung von Erregern ist lediglich beispielhaft und keineswegs beschränkend aufzufassen.

Die Wirksamkeit der erfindungsgemässen 6 α-Methoxypenicilline lässt sich durch folgende Untersuchungen beispielhaft demonstrieren:

1. *In vitro-Versuche*

Für die Untersuchungen wurde die Methode des Reihenverdünnungstextes im Mikrotitersystem angewandt. Die Prüfung der Substanzen auf Bakteriostase erfolgte in flüssigem Medium. Es wurde die Bakteriostasewirkung bei folgenden Konzentrationen untersucht:
128; 64; 32; 16; 8; 4; 2; 1; 0,5; 0,25; 0,12; 0,06 ug/ml. Es wurde ein Nährboden folgender Zusammensetzung benutzt: 10 g Pepton, 8 g Fleischextrakt-Oxoid, 3 g Natriumchlorid, 2 g sek. Natriumphosphat werden mit dest. Wasser auf 100 ml aufgefüllt (pH 7,2-7,4). Das Alter der Primärkulturen betrug etwa 20 Stunden. Die Einstellung der Keimsuspension erfolgte am Photometer (nach «Eppendorf») (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1%ige Barium-chloridlösung in 97 ml 1%ige Schwefelsäure entstand. Nach der Einstellung wurden Streptococcus Aronson im Verhältnis 1:15 und die übrigen Testkeime im Verhältnis 1:1500 mit einer Kochsalzlösung weiter verdünnt.

16 mg der jeweiligen Substanz wurden in 10 ml-Messkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:
Escherichia coli ATCC 11 775, Serratia marcescens ATCC 13 880, Klebsiella pneumoniae ATCC 10 031 und BC 6, Proteus mirabilis BC 17, Proteus rettgeri BC 7, Enterobacter cloacae ATCC 13 047, E. coli $R^+$TEM (β-Lactamase-Träger) und K. pneumoniae 1088 E (β-Lactamase-Träger).

In der folgenden Tabelle 1 sind die ermittelten minimalen Hemmkonzentrationen (MIC) für typische Vertreter der erfindungsgemässen Verbindungen aufgeführt. Es handelt sich um die Natriumsalze von Verbindungen der allgemeinen Formel I mit A = p-Hydroxyphenyl und folgender Bedeutung von R:

| Substanz | R |
|---|---|
| A | 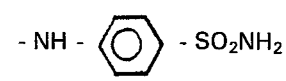 |
| B | 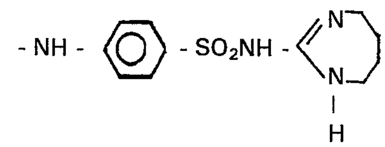 |
| C | 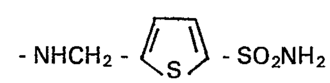 |
| D | 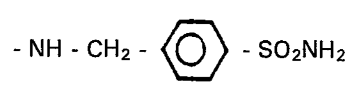 |
| E | Temocillin |

*Tabelle 1:*

| Substanz | E. coli ATCC 11775 | Serr. marc. ATCC 13880 | K. pneum. ATCC 10031 | K. pneum. BC 6 | Prot. mir. BC 17 | Prot. rettg. BC 7 | Eb. cloac. ATCC 13047 | E. coli $R^+$TEM | K. pneum. 1088 E |
|---|---|---|---|---|---|---|---|---|---|
| A | 1 | 1 | 1 | 1 | 1 | 2 | 1 | 1 | 4 |
| B | 0,25 | 0,5 | 0,25 | 0,25 | 0,06 | 0,25 | 0,12 | 0,25 | 1 |
| C | 1 | 2 | 1 | 1 | 0,12 | 2 | 1 | 2 | 8 |
| D | 2 | 2 | 1 | 1 | 0,5 | 4 | 1 | 2 | 8 |
| E | 4 | 8 | 4 | 2 | 2 | 4 | 4 | 8 | 4 |

Um die besonders gute Wirksamkeit einer der erfindungsgemässen 6 α-Methoxypenicilline zu demonstrieren, wurde analog der beschriebenen Methode die in vitro Wirkung von Verbindung A gegen 20 β-Lactamase-tragende Serratia marcesc. im Vergleich zu Temocillin (Verbindung E) geprüft:

*Tabelle 2:*

| Erreger (n) | Inoculum | Geometrisches Mittel (MHK) [Bereich (μg/ml)] A | E |
|---|---|---|---|
| Serr. marc. | $5 \times 10^4$ | 2,8 [2-8] | 10,2 [8-16] |
| (20) | $5 \times 10^6$ | 10,2 [4-32] | 24,3 [16->64] |

Die akute Toxizität wurde durch perorale und subcutane Applikation der Verbindungen der Tabelle 1 und 2 an weissen Laboratoriumsmäusen in steigenden Dosen bestimmt.

Die $LD_{50}$ ist die Dosis, nach deren Applikation 50% der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 2 g/kg, d.h. bei 2 g/kg starben keine Tiere, sie sind damit für die Praxis untoxisch.

Eine Reihe der erfindungsgemässen Verbindungen wurde in vivo bei experimentellen Infektionen an Mäusen untersucht. Man verwendete als pathogene Bakterien E. coli ATCC 11775. Es wurde eine intraperitoneale Infektion mit 0,2 ml einer Bakteriensuspension (mit 5% Mucin) gesetzt. Dies entspricht etwa $1{,}4 \times 10^6$ Keime E. coli/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemässen Penicilline subcutan zur Bestimmung der $ED_{50}$ (Dosis bei der 50% der Tiere überleben) behandelt. Es wurde einmal therapiert (1 Stunde post-Infektionem).

Der Beobachtungszeitraum betrug in beiden Fällen 7 Tage. Die Ergebnisse dieser Teste mit 2 repräsentativen Vertretern der erfindungsgemässen Penicilline sind in der nachfolgenden Tabelle 3 im Vergleich zu Temocillin dargestellt:

*Tabelle 3:*

| Verbindung | E. coli - Infektion $ED_{50}$ (mg/kg) |
|---|---|
| A | 4,0 |
| B | 6,1 |
| E | >20 |

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einen Dosierung zwischen 5 und 500 mg/kg Körpergewicht, vorzugsweise 10-200 mg/kg Körpergewicht, je 24 Stunden verabreicht, gegebenenfalls in Form mehrerer Einzelgaben. Eine Einzelgabe enthält den oder die erfindungsgemässen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die folgenden Beispiele sollen die Erfindung näher erläutern:

### *Beispiel 1*

*Natrium-6β-{D-α-[3-(4-hydroxy-2-p-sulfamoylanilino-5-pyrimidinyl)-ureido]-(phenylacetyl)-amino}-6α-methoxy-penicillansäure*

1,9 g (0,005 Mol) 6α-Methoxy-ampicillin-Monohydrat werden in 100 ml 80%igem wässrigem Tetrahydrofuran suspendiert und mit Triäthylamin unter Eiskühlung in Lösung gebracht. Bei 5°C werden portionsweise 1,55 g 1-Hydro-5-(4-sulfanilamido)-oxazolo-[5,4-d]-pyrimidin-2-on (0,005 Mol) als Festsubstanz zugegeben. Der pH-Wert wird mit Triäthylamin bei 7,5 gehalten. Es wird eine Stunde bei Raumtemperatur gerührt, 30 ml Wasser zugegeben und das Tetrahydrofuran bei 25°C im Vakuum entfernt. Die wässrige Lösung wird bei pH 7,0 zweimal mit Essigester gewaschen und danach unter Eiskühlung mit 1 n Salzsäure auf pH 2,8 gestellt. Der Niederschlag wird abgesaugt und getrocknet. Das Produkt wird mit der äquimolekularen Menge Natrium-2-äthylhexanoat in 30 ml Methanol in Lösung gebracht und

das Natriumsalz sofort durch Zugabe von 200 ml Diäthyläther ausgefällt.
Ausbeute: 1,85 g Natriumsalz (52%);
IR-Spektrum: 1765, 1660, 1600, 1540 $cm^{-1}$;
NMR-Spektrum (DMSO, $CD_3OD$)
Signale bei ppm: 1.0 + 1.25 (2s, 6H), 3.45 (s, 3H), 3.9 (s, 1H), 5.35 (s, 1H), 5.5 (s, 1H), 7.2 - 7.6 (m, 5H), 7.8 (q, 4H), 8.3 (s, 1H).

*Beispiel 2*

*Natrium-6β-{D-α-[3-(4-hydroxy-2-(5'-sulfamoyl-2'--thienylmethylamino)-5-pyrimidinyl)-ureido]--(phenylacetyl)-amino}-6α-methoxy-penicillansäure*

900 mg 5-Amino-4-hydroxy-2-(5'-sulfamoyl-2'--thienylmethylamino)-pyrimidin (0,003 Mol) werden in 50 ml wasserfreiem Tetrahydrofuran suspendiert und nach Zugabe von 5 ml Trimethylsilyldiäthylamin 2,5 Stunden bei Raumtemperatur gerührt.

Anschliessend wird bei 30°C zur Trockne eingedampft und 30 Minuten im Hochvakuum getrocknet. Der Rückstand wird in 50 ml wasserfreiem Tetrahydrofuran aufgenommen und bei 0 bis 5°C zu 3,75 ml einer Phosgenlösung getropft, die 20 g Phosgen in 250 ml wasserfreiem Tetrahydrofuran enthält. Es wird 30 Minuten bei Raumtemperatur gerührt und dann bei maximal 30°C auf ein Volumen von etwa 40 ml eingedampft (Lösung I).

1,145 g (3,1 mMol) 6α-Methoxy-ampicillin-Monohydrat werden in 80 ml 80%igem wässrigem Tetrahydrofuran suspendiert und durch Einstellen des pH-Wertes auf 8,3 (Triäthylamin) in Lösung gebracht. Bei 5 bis 10°C wird Lösung I zugetropft und der pH-Wert durch Triäthylamin-Zugabe bei 7,5 gehalten. Es wird eine Stunde bei Raumtemperatur gerührt, 30 ml Wasser zugegeben und das Tetrahydrofuran bei 30°C im Vakuum entfernt. Die verbleibende wässrige Lösung wird unter Kühlung mit 2N-Salzsäure auf pH 2,7 eingestellt, der Niederschlag wird abgesaugt, mit Wasser gewaschen und getrocknet. Das Produkt wird in Methanol suspendiert und Natrium-2-äthylhexanoat zugegeben. Nach Zugabe von Diäthyläther erhält man 1,03 g (48% der Theorie) der Titelverbindung als farbloses Pulver.
IR-Spektrum: 1765, 1660 $cm^{-1}$;
NMR-Spektrum (DMSO + $CD_3OD$)
Signale bei ppm: 1.0 + 1.25 (2s, 6H), 3.46 (s, 3H), 3.9 (s, 1H), 4.65 (s, 2H), 5.4 (s, 1H), 5.55 (s, 1H), 7.05 (d, 1H), 7.45 (m, 5 + 1H), 8.15 (s, 1H).

Auf analoge Weise wurden die in der folgenden Tabelle zusammengefassten Verbindungen der Formel

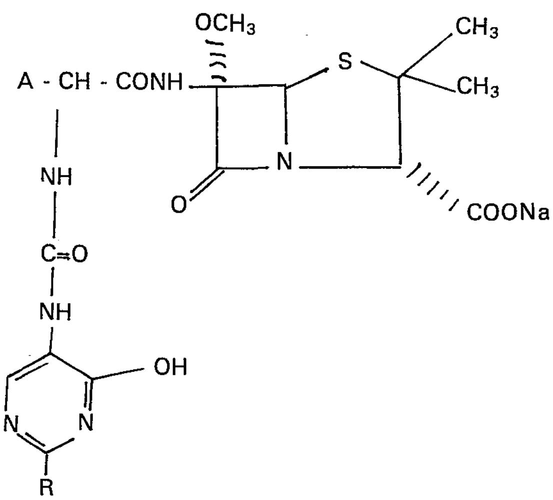

hergestellt.

| | A | R | %-Ausbeute | IR-Spektrum $cm^{-1}$ | NMR-Spektrum (DMSO, $CD_3OD$) Signale bei ppm: |
|---|---|---|---|---|---|
| 3 | HO - 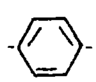 - | - NH - 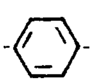 - $SO_2NH_2$ | 57,5 | 1765, 1655 | 1.05 + 1.25 (2s, 6H), 3.4 (s, 3H), 3.9 (s, 1H), 5.35 (2s, 2H), 6.7 (d, 2H), 7.3 (d, 2H), 7.75 (q, 4H), 8.3 (s, 1H) |
| 4 | HO - 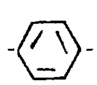 - | - NH - $CH_2$ - 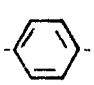 - $SO_2NH_2$ | 44 | 1765, 1660, 1610 | 1.0 + 1.2 (2s, 6H), 3.4 (s, 3H), 3.85 (s, 1H), 4.60 (s, 2H), 5.3 (s, 1H), 5.45 (s, 1H), 6.7 (d, 2H), 7.05 (d, 2H), 7.25 (d, 2H), 7.45 (d, 2H), 8.05 (s, 1H) |
| 5 | 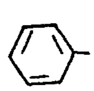 | 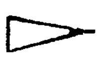 | 61,5 | 1765, 1655 | 0.95 (m, 4H), 1.0 + 1.3 (2s, 6H), 1.85 (m, 1H), 3.4 (s, 3H), 3.9 (s, 1H), 5.35 (2s, 2H), 7.5 (m, 5H), 8.35 (s, 1H) |
| 6 | 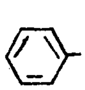 | - NH - 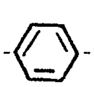 - $SO_2CH_3$ | 50 | 1765, 1660 | 1.0 + 1.3 (2s, 6H), 3.1 (s, 3H), 3.4 (s, 3H), 3.9 (s, 1H), 5.3 (s, 1H), 5.55 (s, 1H), 7.2 - 7.6 (m, 5H), 7.9 (q, 4H), 8.3 (s, 1H) |

| | A | R | %-Ausbeute | IR-Spektrum $cm^{-1}$ | NMR-Spektrum (DMSO, $CD_3OD$) Signale bei ppm: |
|---|---|---|---|---|---|
| 7 | 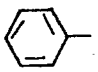 | 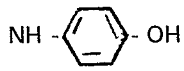 | 39 | 1765, 1660 | 1.0 + 1.3 (2s, 6H), 3.4 (s, 3H), 3.85 (s, 1H), 5.3 (2s, 2H), 6.9 (d, 2H), 7.45 (m, 7H), 8.10 (s, 1H) |
| 8 | 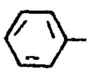 | 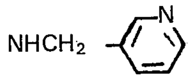 | 61,5 | 1765, 1660, 1605 | 1.0 + 1.3 (2s, 6H), 3.4 (s, 3H), 3.90 (s, 1H), 4.30 (s, 2H), 5.35 (s, 1H), 5.5 (s, 1H), 7.25 (m, 1H), 7.45 (m, 5H), 7.7 (m, 1H), 8.1 (s, 1H), 8.45 (m, 2H) |
| 9 | 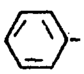 | 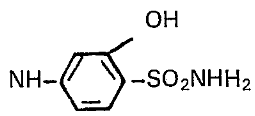 | 53 | 1765, 1660, 1610 | 0.95 + 1.25 (2s, 6H), 3.4 (s, 3H), 3.85 (s, 1H), 5.3 (s, 1H), 5.5 (s, 1H), 7.0 (m, 1H), 7.2 - - 7.7 (m, 7H), 8.3 (s, 1H) |
| 10 | 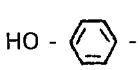 | 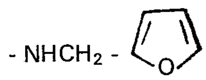 | 55 | 1765, 1655 | 1.0 + 1.2 (2s, 6H), 3.4 (s, 3H), 3.8 (s, 1H), 4.35 (s, 2H), 5.25 (2s, 1H), 6.25 (m, 2H), 6.65 (d, 2H), 7.2 (d, 2H), 7.45 (m, 1H), 8.0 (s, 1H) |
| 11 | 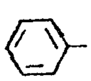 | 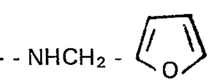 | 58,5 | 1765, 1655 | 1.0 + 1.3 (2s, 6H), 3.4 (s, 3H), 3.90 (s, 1H), 4.40 (s, 2H), 5.35 (s, 1H), 5.5 (s, 1H), 6.3 (m, 2H), 7.2 - 7.6 (m, 5 + 1H), 8.05 (s, 1H) |
| 12 | 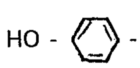 | 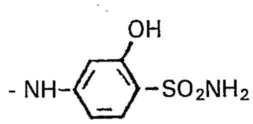 | 69 | 1765, 1655 | 1.05 + 1.3 (2s, 6H), 3.4 (s, 3H), 3.85 (s, 1H), 5.35 (2s, 2H), 6.7 (d, 2H), 7.0 - 7.6 (m, 4H), 7.7 (s, 1H), 8.3 (s, 1H) |
| 13 | 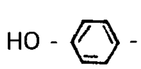 | 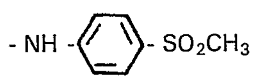 | 47 | 1765, 1655 | 1.0 + 1.25 (2s, 6H), 3.1 (s, 3H), 3.4 (s, 3H), 3.85 (s, 1H), 5.35 (2s, 2H), 6.7 (d, 2H), 7.3 (d, 2H), 7.7 (d, 2H), 8.0 (d, 2H), 8.3 (s, 1H) |
| 14 | 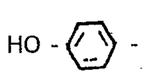 | 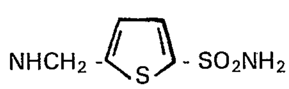 | 64 | 1765, 1660 | 1.0 + 1.25 (2s, 6H), 3.4 (s, 3H), 3.85 (s, 1H), 4.6 (s, 2H), 5.3 (2s, 2H), 6.65 (d, 2H), 6.9 (d, 1H), 7.25 (d + d, 1 + 2H), 8.0 (s, 1H) |
| 15 | 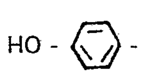 | 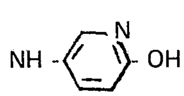 | 59 | 1765, 1655 | 1.0 + 1.3 (2s, 6H), 3.45 (s, 3H), 3.85 (s, 1H), 5.35 (2s, 2H), 6.35 (d, 1H), 6.7 (d, 2H), 7.3 (d, 2H), 7.5 (dd, 1H), 7.9 (d, 1H), 8.15 (s, 1H) |
| 16 | 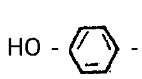 | 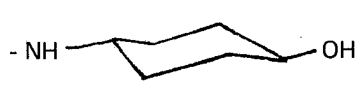 | 47 | 1765, 1655 | 1.0 (s, 3H), 1.2 (m, 4H), 1.25 (s, 3H), 1.85 (m, 2H), 3.4 (s, 3H), 3.42 (m, 1H), 3.55 (m, 1H), 3.85 (s, 1H), 5.32 (2s, 2H), 6.7 (d, 2H), 7.3 (d, 2H), 8.0 (s, 1H) |

| | A | R | %-Ausbeute | IR-Spektrum $cm^{-1}$ | NMR-Spektrum (DMSO, $CD_3OD$) Signale bei ppm: |
|---|---|---|---|---|---|
| 17 | 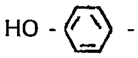 | - $NHCH_3H_7$ | 72 | 1765, 1660 | 0.85 (t, 3H), 1.0 + 1.3 (2s, 6H), 1.5 (q, 2H), 3.4 (s, 3H), 3.9 (s, 1H), 5.35 (2s, 2H), 6.7 (d, 2H), 7.3 (d, 2H), 8.0 (s, 1H) |
| 18 | 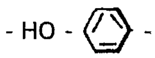 | 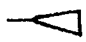 | 86 | 1765, 1660 | 0.95 (m, 4H), 1.0 + 1.3 (2s, 6H), 1.85 (m, 1H), 3.4 (s, 3H), 3.9 (s, 1H), 5.3 (2s, 2H), 6.7 (d, 2H), 7.3 (d, 2H), 8.35 (s, 1H) |
| 19 | 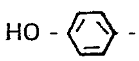 | 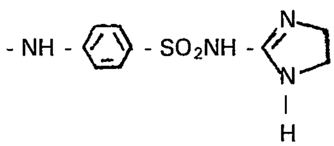 | 45 | 1765, 1660 | 1.05 + 1.3 (2s, 6H), 3.15 (m, 4H), 3.4 (s, 3H), 3.85 (s, 1H), 5.3 (2s, 2H), 6.7 (d, 2H), 7.3 (d, 2H), 7.6 (d, 2H), 7.85 (d, 2H), 8.25 (s, 1H) |
| 20 | 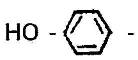 | 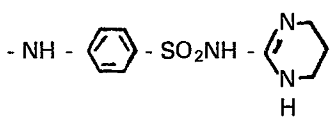 | 58 | 1765, 1655 | 1.05 + 1.3 (2s, 6H), 1.8 (m, 2H), 3.15 (m, 4H), 3.4 (s, 3H), 3.85 (s, 1H), 5.35 (2s, 2H), 6.7 (d, 2H), 7.3 (d, 2H), 7.6 (d, 2H), 7.85 (d, 2H), 8.25 (s, 1H) |
| 21 | 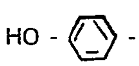 | 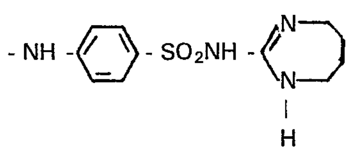 | 61 | 1765, 1660 | 1.0 + 1.3 (2s, 6H), 1.55 (m, 4H), 3.05 (m, 4H), 3.4 (s, 3H), 3.9 (s, 1H), 5.35 (2s, 2H), 6.75 (d, 2H), 7.35 (d, 2H), 7.65 (d, 2H), 7.9 (d, 2H), 8.35 (s, 1H) |
| 22 | 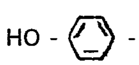 | 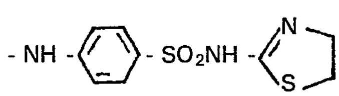 | 70 | 1765, 1660 | 1.05 + 1.3 (2s, 6H), 2.5 (m, 2H), 3.2 (m, 2H), 3.4 (s, 3H), 3.9 (s, 1H), 5.35 (2s, 2H), 6.7 (d, 2H), 7.3 (d, 2H), 7.6 (d, 2H), 7.9 (d, 2H), 8.25 (s, 1H) |

*Darstellung pharmazeutischer Anwendungsformen*

Die Verbindungen der allgemeinen Formel I und I' lassen sich in die üblichen pharmazeutischen Anwendungsformen, wie Tabletten, Dragees, Kapseln oder Ampullen einarbeiten. Die Einzeldosis beträgt bei Erwachsenen im allgemeinen zwischen 50 und 800 mg, vorzugsweise 200 bis 500 mg, die Tagesdosis zwischen 150 und 2 500 mg, vorzugsweise 600 bis 1 500 mg.

*Beispiel I*

*Tabletten enthaltend Natrium-6β-{D-α-[3-(4-hydroxy-2-p-sulfamoylanilino-5-pyrimidinyl)-ureido]-benzylamido}-6α-methoxy-penicillansäure*

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepresst, derart, dass jede Tablette 200 mg Wirkstoff enthält.

*Beispiel II*

*Dragees enthaltend Natrium-6β-{D-α-[3-(4-hydroxy-2-p-sulfamoylanilino-5-pyrimidinyl)-ureido]-benzylamido}-6α-methoxy-penicillansäure*

Analog Beispiel I werden Tabletten gepresst, die anschliessend in üblicher Weise mit einem Überzug, bestehend aus Zucker, Kartoffelstärke, Talk und Tragant überzogen werden.

*Beispiel III*

*Kapseln enthaltend Natrium-6β-{D-α-[3-(4-hydroxy-2-p-sulfamoylanilino-5-pyrimidinyl)-ureido]-benzylamido}-6α-methoxy-penicillansäure*

5 kg Wirksubstanz werden in üblicher Weise in Hartgelatinekapseln gefüllt, derart, dass jede Kapsel 500 mg des Wirkstoffes enthält.

*Beispiel IV*

*Trockenampullen enthaltend Natrium-6β-{D-α-[3-(4-hydroxy-2-p-sulfamoylanilino-5-pyrimidinyl)-ureido]-benzylamido}-6α-methoxy-penicillansäure*

In einem aseptischen Bereich wurde 251 g Wirkstoff in 2008 ml destilliertem Wasser zur Injektion aufgelöst. Die Lösung wurde durch ein Millipore-Filter (Porengrösse 0,22 um, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragees, Kapseln und Ampullen erhältlich, die einen oder mehrere der übrigen Wirkstoffe der Formel I oder die physiologisch unbedenklichen Salze dieser Verbindungen enthalten.

Ebenso können Trockenampullen hergestellt werden, die als Wirkstoff Natrium-6β-{D-α-[3-(4-hydroxy-2-p-sulfamoylanilino-5-pyrimidinyl)-ureido]-benzylamido}-6α-methoxy-penicillansäure enthalten.

**Patentansprüche für die Vertragsstaaten:**
BE, CH, DE, FR, IT, LI, LU, NL, SE

1. 6 α-Methoxy-penicilline der allgemeinen Formel

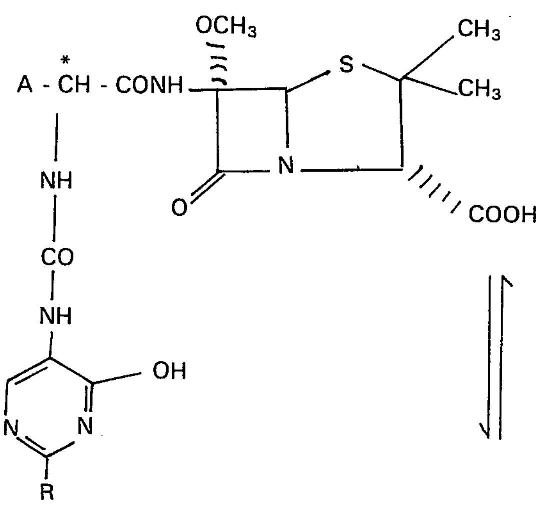
,(I)

bzw.

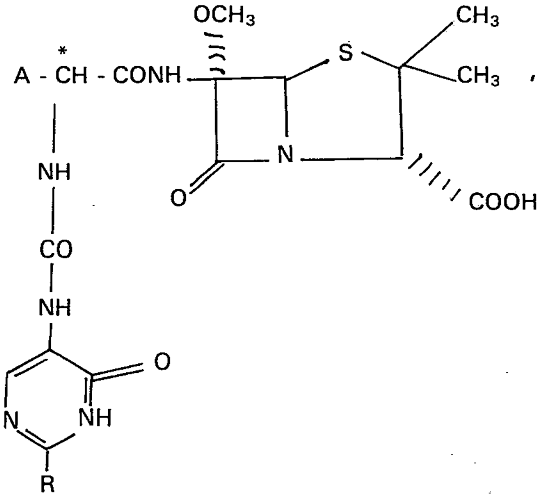
, (I')

in der

A die Phenyl-, p-Hydroxyphenyl-, 2- oder 3-Thienylgruppe,

R die Cyclopropylgruppe, eine Gruppe der allgemeinen Formel

- NH - $R_1$, wobei

$R_1$ einen gegebenenfalls ab Kohlenstoffatom 2 durch eine Hydroxygruppe substituierten aliphatischen, verzweigten oder unverzweigten Kohlenwasserstoffrest mit 1 bis 4 (ausser 1 und 2) Kohlenstoffatomen, einen gegebenenfalls durch eine Hydroxygruppe substituierten Cycloalkylrest mit 3 bis 6 Kohlenstoffatomen oder eine gegebenenfalls durch eine Hydroxy-, Methylsulfinyl-, Methylsulfonyl- oder Aminosulfonylgruppe substituierte 3-Pyridyl-, 2-Furylmethyl-, 2-Thienylmethyl-, 3-Imidazolylmethyl-, 2-Thiazolylmethyl- oder 3-Pyridylmethylgruppe darstellt,

eine Gruppe der allgemeinen Formel

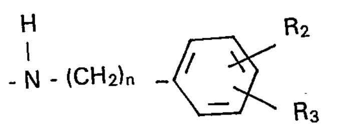
, wobei

n die Zahl 0 oder 1,

$R_2$ und $R_3$, die gleich oder verschieden sein können Wasserstoffatome, Hydroxy-, Acetylamino-, Aminocarbonylamino-, Nitro-, Aminocarbonyl-, Cyan-, Methylsulfinyl-, Methylsulfonyl-, Aminosulfonyl-, Methylaminosulfonyl-, Aminocarbonylmethylenaminosulfonyl-, 2'-Hydroxyäthylaminosulfonyl-, Cyanaminosulfonyl-, Aminocarbonylaminosulfonyl-, Acetylaminosulfonyl-, Methylsulfonylaminosulfonyl- oder Acetylhydrazinosulfonylgruppen darstellen,

eine Gruppe der allgemeinen Formel

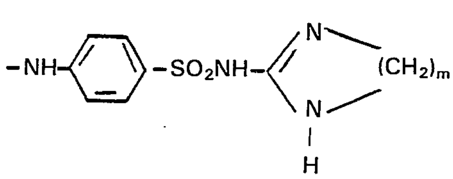
, wobei

m die Zahl 2, 3 oder 4 darstellt, oder eine Gruppe der Formel

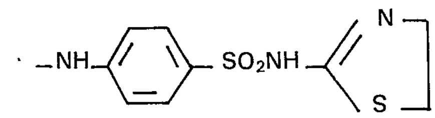

bedeuten und deren Salze mit organischen oder anorganischen Basen.

2. 6 α-Methoxy-penicilline der allgemeinen Formel I bzw. I' gemäss Anspruch 1, dadurch gekennzeichnet, dass R die p-Aminosulfonylanilino-, p-Methylsulfinylanilino-, p-Methylsulfonylanilino-, m-Hydroxy-p-aminosulfonylanilino-, p-Aminocarbonylmethylenamino-sulfonylanilino-, p-(4',5'-Dihydro-imidazol-2'-yl)-aminosulfonylanilino-, p-(3',4',5',6'-Tetrahydro-pyrimidin-2'-yl)-aminosulfonylanilino-, p-(4',5',6',7'-Tetrahydro-1,3-diazepin-2'-yl)-aminosulfonylanilino-, p-(4',5'-Dihydro-thiazol-2'-yl)-aminosulfonylanilino-, p-Hydroxybenzylamino-, p-Aminosulfonylbenzylamino-, Isopropylamino-, 4'-Hydroxycyclohexylamino-, 5'-Aminosulfonyl-2'-thienylmethylamino-, 2'-Furylmethylamino-, 3'-Pyridylmethylamino- oder 4'-Hydroxy-3'-pyridylaminogruppe bedeutet und A wie im Anspruch 1 definiert ist, und ihre Salze mit anorganischen oder organischen Basen.

3. 6 α-Methoxy-penicilline der allgemeinen Formel I bzw. I' gemäss Anspruch 1 und 2, dadurch gekennzeichnet, dass diese in D = R-Konfiguration vorliegen.

4. 6β-{D-α-[3-(4-Hydroxy-2-p-(4',5',6',7'-tetrahydro-1,3-diazepin-2'-yl)-aminosulfonylanilino-5-pyrimidinyl)-ureido]-p-hydroxy-benzylamido}-6α-methoxy-penicillansäure und dessen Natriumsalz.

5. 6β-{D-α-[3-(4-Hydroxy-2-p-sulfamoylanilino-5-pyrimidinyl)-ureido]-p-hydroxybenzylamido}-6α-methoxy-penicillansäure und dessen Natriumsalz.

6. Arzneimittel gekennzeichnet durch den Gehalt an einem oder mehreren Wirkstoffen gemäss den Ansprüchen 1 bis 5 neben den üblichen Träger- und/oder Hilfsstoffen.

7. Verfahren zur Herstellung von Verbindungen gemäss Anspruch 1, dadurch gekennzeichnet, dass eine Verbindung der allgemeinen Formel II

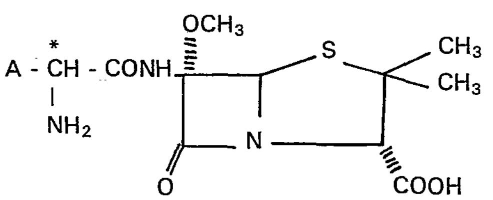
, (II)

in der A wie in Anspruch 1 definiert ist, mit einem gegebenenfalls in Reaktionsgemisch hergestellten Pyrimidinderivat der allgemeinen Formel III

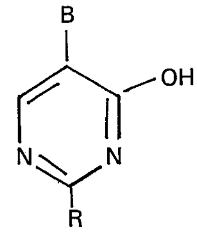
, (III)

oder

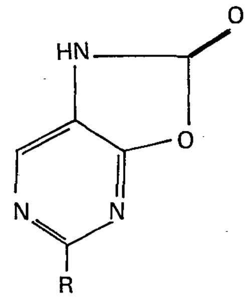
, (IIIa)

in denen

R wie in Anspruch 1 definiert ist und

B die Gruppe -NCO oder ein reaktives Derivat der Gruppe NHCOOH, wie z.B. die Gruppen -NHCOCl, -NHCOBr oder -NH-COO- 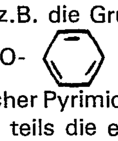 -$NO_2$, bedeutet,

oder mit Gemischen solcher Pyrimidine der allgemeinen Formel III, in der B teils die eine und teils die andere der vorstehend genannten Bedeutungen besitzt, in einem Lösungsmittel und in einem pH-Bereich zwischen 2,0 und 9,0 bei Temperaturen zwischen —20°C und +50°C umgesetzt wird, und eine so erhaltene Verbindung der allgemeinen Formel I bzw. I' anschliessend, falls erwünscht, in ihre physiologisch verträglichen Salze mit anorganischen oder organischen Basen überführt wird.

8. Verfahren gemäss Anspruch 7, dadurch gekennzeichnet, dass eine Verbindung der allgemeinen Formel II oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0 oder

b) in wasserfreien Lösungsmitteln oder

c) in einem Gemenge aus Wasser und mit Wasser nicht mischbaren Lösungsmitteln in einem pH-Bereich zwischen 6,5 und 8,0

umgesetzt wird, oder

dass eine Verbindung der allgemeinen Formel II, in der das Wasserstoffatom der Carboxylgruppe durch eine Silylgruppe oder eine andere leicht abspaltbare Schutzgruppe ersetzt ist, mit einer Verbindung der allgemeinen Formel III in einem wasser- und hydroxylgruppenfreien bzw. aprotischen Lösungsmittel, gegebenenfalls in Gegenwart einer Base, umgesetzt wird.

**Patentansprüche für den Vertragsstaat:** AT

1. Verfahren zur Herstellung von 6 α-Methoxy-penicillinen der allgemeinen Formel

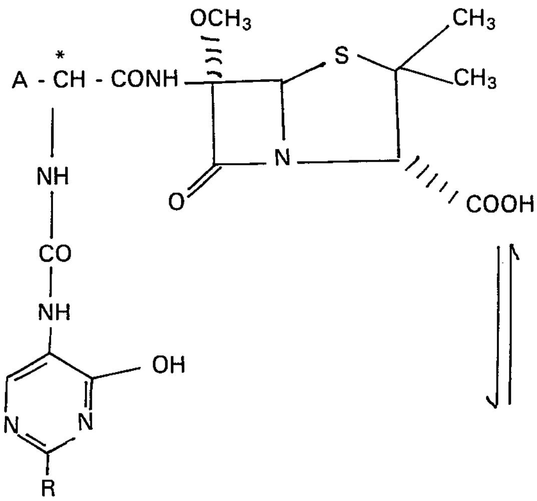

,(I)

bzw.

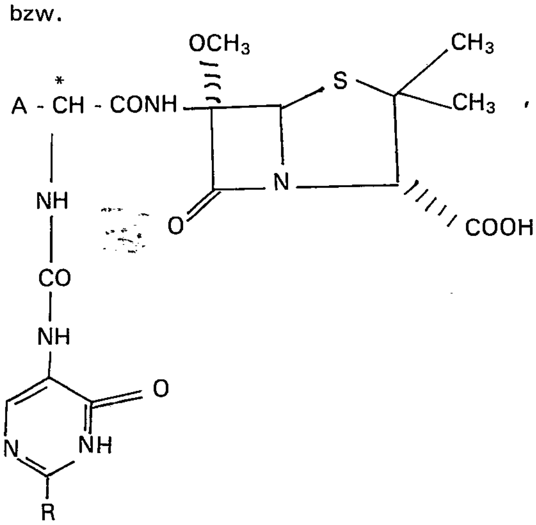

, (I')

in der

A die Phenyl-, p-Hydroxyphenyl-, 2- oder 3-Thienylgruppe;

R die Cyclopropylgruppe, eine Gruppe der allgemeinen Formel

$- NH - R_1,$ wobei

$R_1$ einen gegebenenfalls ab Kohlenstoffatom 2 durch eine Hydroxygruppe substituierten aliphatischen, verzweigten oder unverzweigten Kohlenwasserstoffrest mit 1 bis 4 Kohlenstoffatomen, einen gegebenenfalls durch eine Hydroxygruppe substituierten Cycloalkylrest mit 3 bis 6 Kohlenstoffatomen oder eine gegebenenfalls durch eine Hydroxy-, Methylsulfinyl-, Methylsulfonyl- oder Aminosulfonylgruppe substituierte 3-Pyridyl-, 2-Furylmethyl-, 2-Thienylmethyl-, 3-Imidazolylmethyl-, 2-Thiazolylmethyl- oder 3-Pyridylmethylgruppe darstellt,

eine Gruppe der allgemeinen Formel

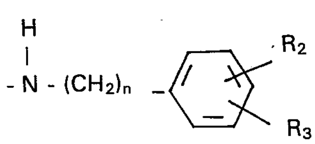

, wobei

n die Zahl 0 oder 1,

$R_2$ und $R_3$, die gleich oder verschieden sein können Wasserstoffatome, Hydroxy-, Acetylamino-, Aminocarbonylamino-, Nitro-, Aminocarbonyl-, Cyan-, Methylsulfinyl-, Methylsulfonyl-, Aminosulfonyl-, Methylaminosulfonyl-, Aminocarbonylmethylenaminosulfonyl-, 2'-Hydroxyäthylaminosulfonyl-, Cyanaminosulfonyl-, Aminocarbonylaminosulfonyl-, Acetylaminosulfonyl-, Methylsulfonylaminosulfonyl- oder Acetylhydrazinosulfonylgruppen darstellen,

eine Gruppe der allgemeinen Formel

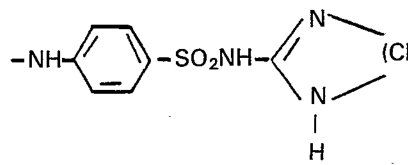

, wobei

m die Zahl 2, 3 oder 4 darstellt, oder

eine Gruppe der Formel

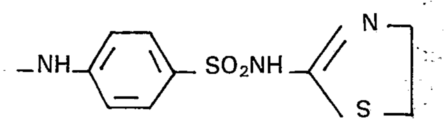

bedeuten, und von deren Salzen mit anorganischen oder organischen Basen, dadurch gekennzeichnet, dass

eine Verbindung der allgemeinen Formel II

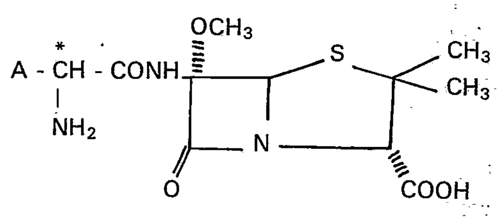

, (II)

in der A wie oben definiert ist, mit einem gegebenenfalls in Reaktionsgemisch hergestellten Pyrimidinderivat der allgemeinen Formel III

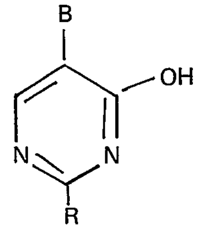

, (III)

oder

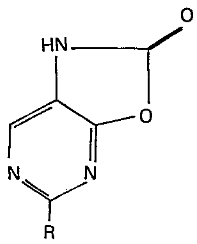

, (IIIa)

in denen

R wie oben definiert ist und

B die Gruppe -NCO oder ein reaktives Derivat der Gruppe NHCOOH, wie z.B. die Gruppen -NHCOCl, -NHCOBr oder -NH-COO- 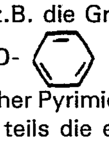 $-NO_2$, bedeutet,

oder mit Gemischen solcher Pyrimidine der allgemeinen Formel III, in der B teils die eine und teils die andere der vorstehend genannten Bedeutungen besitzt, in einem Lösungsmittel und in einem pH-Bereich zwischen 2,0 und 9,0 bei Temperaturen zwischen —20°C und +50°C umgesetzt wird, und eine so erhaltene Verbindung der allgemeinen Formel I bzw. I' anschliessend, falls erwünscht, in ihre physiologisch verträglichen Salze mit anorganischen oder organischen Basen überführt wird.

2. Verfahren gemäss Anspruch 1, dadurch gekennzeichnet, dass eine Verbindung der allgemeinen Formel II oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0 oder

b) in wasserfreien Lösungsmitteln oder

c) in einem Gemenge aus Wasser und mit Wasser nicht mischbaren Lösungsmitteln in einem pH-Bereich zwischen 6,5 und 8,0

umgesetzt wird, oder

dass eine Verbindung der allgemeinen Formel II, in der das Wasserstoffatom der Carboxylgruppe durch eine Silylgruppe oder eine andere leicht abspaltbare Schutzgruppe ersetzt ist, mit einer Verbindung der allgemeinen Formel III in einem wasser- und hydroxylgruppenfreien bzw. aprotischen Lösungsmittel, gegebenenfalls in Gegenwart einer Base, umgesetzt wird.

**Claims for the Contracting States:**
BE, CH, DE, FR, IT, LI, LU, NL, SE

1. 6 α-Methoxy-penicillins of general formula

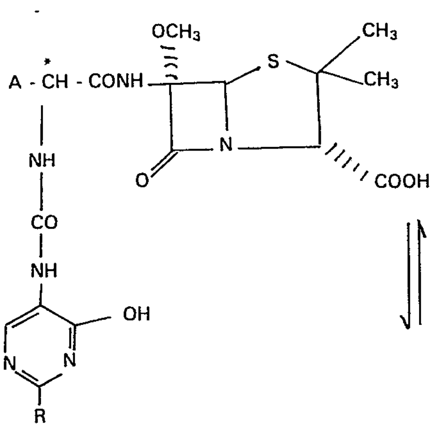

,(I)

or

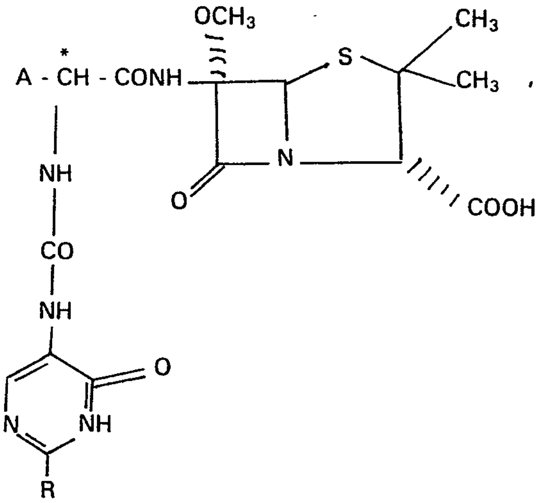

, (I')

wherein

A represents the phenyl, p-hydroxyphenyl, 2- or 3-thienyl group,

R represents the cyclopropyl group, or a group of general formula

$$-NH-R_1,$$

(wherein $R_1$ represents a branched or unbranched alipatic hydrocarbon group with 1 to 4 (with the exception of 1 and 2) carbon atoms, optionally substituted from carbon atom 2 by a hydroxy group; a cycloalkyl group with 3 to 6 carbon atoms optionally substituted by a hydroxy group; or a 3-pyridyl, 2-furylmethyl, 2-thienylmethyl, 3-imidazolylmethyl, 2-thiazolylmethyl or 3-pyridylmethyl group optionally substituted by a hydroxy, methylsulphinyl, methylsulphonyl or aminosulphonyl group),

or a group of general formula

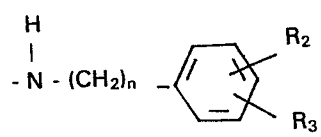

(wherein n represents the number 0 or 1,

$R_2$ and $R_3$, which may be the same or different, may represent hydrogen atoms, hydroxy, acetylamino, aminocarbonylamino, nitro, aminocarbonyl, cyano, methylsulphinyl, methylsulphonyl, aminosulphonyl, methylaminosulphonyl, aminocarbonylmethyleneaminosulphonyl, 2'-hydroxyethylaminosulphonyl, cyanoaminosulphonyl, aminocarbonylaminosulphonyl, acetylaminosulphonyl, methylsulphonylaminosulphonyl or acetylhydrazinosulphonyl groups),

or a group of general formula

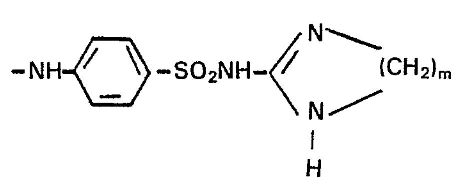

(wherein m represents the number 2, 3 or 4), or a group of formula

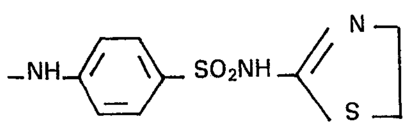

and the salts thereof with inorganic or organic bases.

2. 6 $\alpha$-Methoxy-penicillins of general formula I or I' as claimed in claim 1, characterised in that R represents the p-aminosulphonylanilino, p-methylsulphinylanilino, p-methylsulphonylanilino, m-hydroxy-p-aminosulphonylanilino, p-aminocarbonylmethyleneaminosulphonylanilino, p-(4',5'-dihydro-imidazol-2'-yl)-aminosulphonylanilino, p-(3',4',5',6'-tetrahydropyrimidin-2'-yl)-aminosulphonyl-anilino, p-(4',5',6',7'-tetrahydro-1,3-diazepin-2'-yl)-aminosulphonyl-anilino, p-(4',5'-dihydro-thiazol-2'-yl)-aminosulphonylanilino, p-hydroxybenzylamino, p-aminosulphonylbenzylamino, isopropylamino, 4'-hydroxycyclohexylamino, 5'-aminosulphonyl-2'-thienylmethylamino, 2'-furylmethylamino, 3'-pyridylmethylamino or 4'-hydroxy-3'-pyridylamino group, and A is defined as in claim 1 and the salts thereof with inorganic or organic bases.

3. 6 $\alpha$-Methoxy-penicillins of general formula I or I' as claimed in claims 1 and 2, characterised in that they are in the D = R configuration.

4. 6$\beta$-{D-$\alpha$-[3-(4-hydroxy-2-p-(4',5',6',7'-tetrahydro-1,3-diazepin-2'-yl)-aminosulfonylanilino-5-pyrimidinyl)-ureido]-p-hydroxy-benzylamido}-6$\alpha$-methoxy-penicillanic acid and the sodium salt thereof.

5. 6$\beta$-{D-$\alpha$-[3-(4-hydroxy-2-p-sulphamoylanilino-5-pyrimidinyl)-ureido]-p-hydroxybenzylamido}-6$\alpha$-methoxy-penicillanic acid and the sodium salt thereof.

6. Pharmaceutical compositions, characterised in that they contain one or more active substances as claimed in claims 1 to 5, together with the usual carriers and/or excipients.

7. Process for the preparation of compounds as claimed in claim 1, characterised in that a compound of general formula II

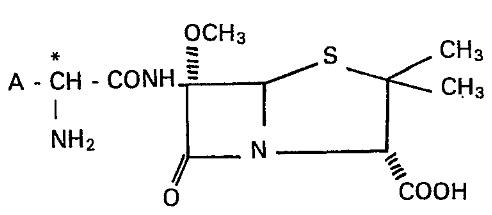
, (II)

wherein A is defined as in claim 1, is reacted with a pyrimidine derivative of general formula

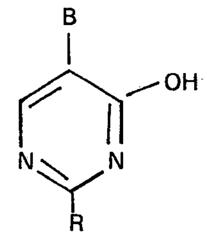
, (III)

or

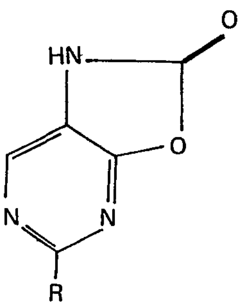
, (IIIa)

optionally prepared in the reaction mixture, wherein R is defined as in claim 1 and B represents the group -NCO or a reactive derivative of the group NHCOOH, such as, for example, the groups -NHCOCl, -NHCOBr or -NH-COO- 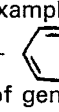 $-NO_2$, or with mixtures of pyrimidines of general formula III wherein B is defined partly according to the first and partly according to the second of the above definitions, in a solvent at a pH of between 2.0 and 9.0 at temperatures between —20 and +50°C, and, if desired, a compound of general formula I or I' thus obtained is subsequently converted into the physiologically acceptable salts thereof with inorganic or organic bases.

8. Process as claimed in claim 7, characterised in that a compound of general formula II or one of its salts with inorganic or organic bases is reacted with a compound of general formula III

a) in water or in water-miscible solvents in the presence of water in a pH range of from 6.5 to 8.0 or

b) in anhydrous solvents or

c) in a mixture of water and water-immiscible solvents in a pH range of between 6.5 and 8.0,

or a compound of general formula II, wherein the hydrogen atom in the carboxyl group is replaced by a silyl group or another protecting group which is easily split off, is reacted with a compound of general formula III in an anhydrous solvent free from hydroxyl groups or an aprotic solvent, optionally in the presence of a base.

**Claims for the Contracting State:** AT

1. Process for the preparation of 6$\alpha$-methoxy-penicillins of general formula

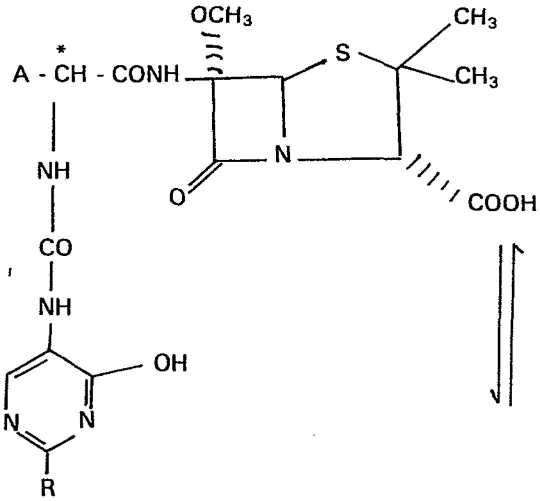

,(I)

or

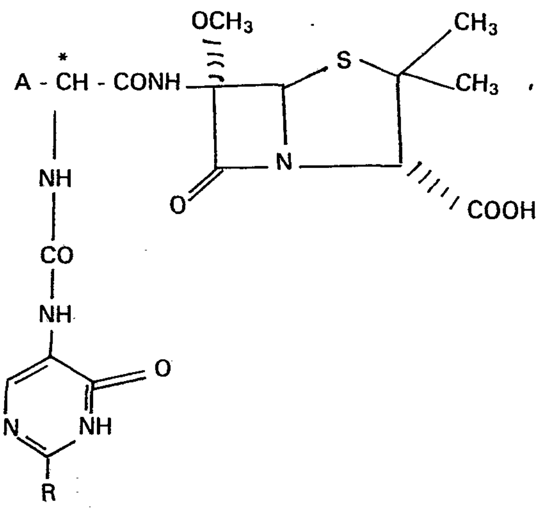

, (I')

wherein

A represents the phenyl, p-hydroxyphenyl, 2- or 3-thienyl group,

R represents the cyclopropyl group, or a group of general formula

$- NH - R_1$,

(wherein $R_1$ represents a branched or unbranched aliphatic hydrocarbon group with 1 to 4 carbon atoms, optionally substituted from carbon atom 2 by a hydroxy group; a cycloalkyl group with 3 to 6 carbon atoms optionally substituted by a hydroxy group; or a 3-pyridyl, 2-furylmethyl, 2-thienylmethyl, 3-imidazolylmethyl, 2-thiazolylmethyl or 3-pyridylmethyl group optionally substituted by a hydroxy, methylsulphinyl, methylsulphonyl or aminosulphonyl group),

or a group of general formula

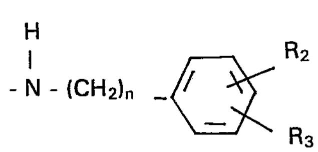

(wherein n represents the number 0 or 1,

$R_2$ and $R_3$, which may be the same or different, may represent hydrogen atoms, hydroxy, acetylamino, aminocarbonylamino, nitro, aminocarbonyl, cyano, methylsulphinyl, methylsulphonyl, aminosulphonyl, methylaminosulphonyl, aminocarbonylmethyleneaminosulphonyl, 2'-hydroxyethylaminosulphonyl, cyanoaminosulphonyl, aminocarbonylaminosulphonyl, acetylaminosulphonyl, methylsulphonylaminosulphonyl or acetylhydrazinosulphonyl groups),

or a group of general formula

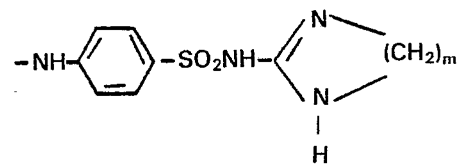

(wherein m represents the number 2, 3 or 4), or a group of formula

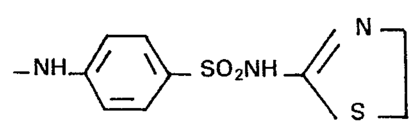

and the salts thereof with inorganic or organic bases, characterised in that a compound of general formula

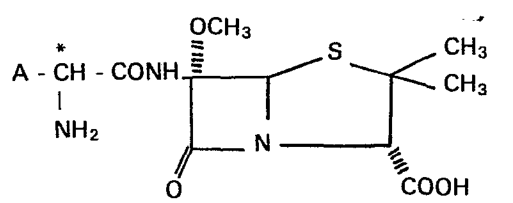

, (II)

wherein A is as hereinbefore defined, is reacted with a pyrimidine derivative of general formula III

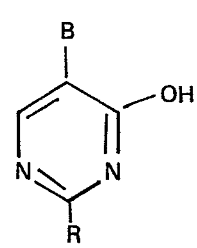

, (III)

or

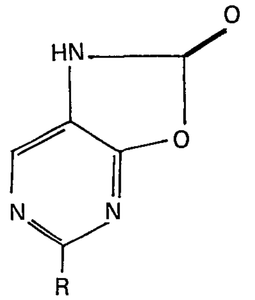

, (IIIa)

optionally prepared in the reaction mixture, wherein R is as hereinbefore defined and B represents the group -NCO or a reactive derivative of the group NHCOOH, such as, for example, the groups -NHCOCl, -NHCOBr or -NH-COO- 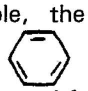 $-NO_2$, or with mixtures of pyrimidines of general formula III wherein B is defined partly according to the first and partly according to the second of the above definitions, in a solvent at a pH of between 2.0 and 9.0 at temperatures between —20 and +50°C, and, if desired, a compound of general formula I or I' thus obtained is subsequently converted into the physiologically acceptable salts thereof with inorganic or organic bases.

2. Process as claimed in claim 1, characterised in that a compound of general formula II or one of its salts with inorganic or organic bases is reacted with a compound of general formula III

a) in water or in water-miscible solvents in the presence of water in a pH range of from 6.5 to 8.0 or

b) in anhydrous solvents or

c) in a mixture of water and water-immiscible solvents in a pH range of between 6.5 and 8.0,

or a compound of general formula II, wherein the hydrogen atom in the carboxyl group is replaced by a silyl group or another protecting group which is easily split off, is reacted with a compound of general formula III in an anhydrous solvent free from hydroxyl groups or an aprotic solvent, optionally in the presence of a base.

**Revendications pour les Etats contractants:**
BE, CH, DE, FR, IT, LI, LU, NL, SE

1. 6-alpha-méthoxy-pénicillines de formule générale:

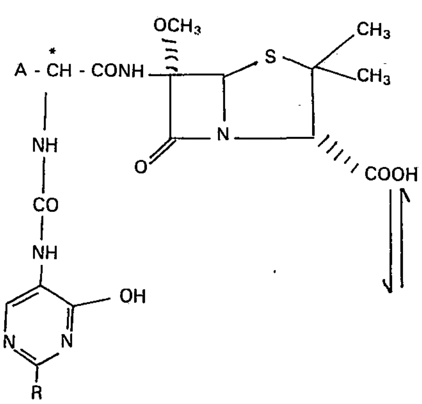

,(I)

ou

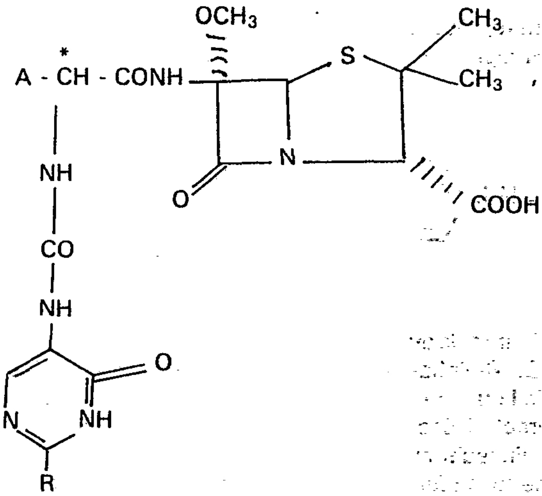

, (I')

dans laquelle:

A représente le groupe phényle, p-hydroxyphényle, 2- ou 3-thiényle,

R représente le groupe cyclopropyle, un groupe de formule générale

$$- NH - R_1, \quad \text{où:}$$

$R_1$ représente un radical hydrocarboné avec 1 à 4 (à l'exception de 1 et de 2) atomes de carbone, aliphatique, ramifié ou non ramifié, éventuellement substitué par un groupe hydroxy à partir de l'atome de carbone 2, ou représente un radical cycloalcoyle avec 3 à 6 atomes de carbone éventuellement substitué par un groupe hydroxy, ou représente un groupe 3-pyridyle, 2-furylméthyle, 2-thiénylméthyle, 3-imidazolylméthyle, 2-thiazolylméthyle ou 3-pyridylméthyle éventuellement substitué par un groupe hydroxy, méthylsulfinyle, méthylsulfonyle ou aminosulfonyle,

ou R représente un groupe de formule générale:

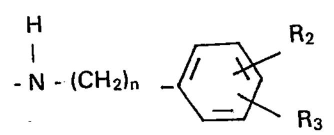

, où

n représente le chiffre 0 ou 1,

$R_2$ et $R_3$ qui peuvent être identiques ou différents représentent des atomes d'hydrogène, des groupes hydroxy, acétylamino, aminocarbonylamino, nitro, aminocarbonyle, cyano, méthylsulfinyle, méthylsulfonyle, aminosulfonyle, méthylaminosulfonyle, aminocarbonylméthylèneaminosulfonyle, 2'-hydroxyéthylaminosulfonyle, cyanaminosulfonyle, aminocarbonylaminosulfonyle, acétylaminosulfonyle, méthylsulfonylaminosulfonyle ou acétylhydrazinosulfonyle,

ou R représente un groupe de formule générale:

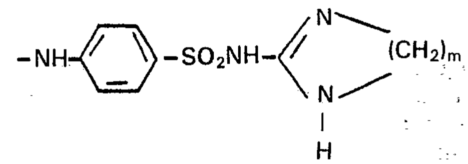

, où

m représente le nombre 2, 3 ou 4, ou
R représente un groupe de formule :

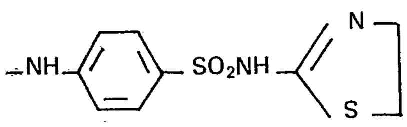

et leurs sels avec des bases organiques ou minérales.

2. 6-alpha-méthoxy-pénicillines de formule générale I ou respectivement I', selon la revendication 1, caractérisées en ce que R représente le groupe p-aminosulfonylanilino, p-méthylsulfinylanilino, p-méthylsulfonylanilino, m-hydroxy-p-aminosulfonylanilino, p-aminocarbonylméthylèneaminosulfonylanilino, p-(4',5'-dihydro-imidazol-2'-yl)-aminosulfonylanilino, p-(3',4',5',6'-tétrahydro-pyrimidine-2'-yl)-aminosulfonylanilino, p-(4',5',6',7'-tétrahydro-1,3-diazépine-2'-yl)-aminosulfonylanilino, p-(4',5'-dihydro-thiazol-2'-yl)-aminosulfonylanilino, p-hydroxybenzylamino, p-aminosulfonylbenzylamino, isopropylamino, 4'-hydroxycyclohexylamino, 5'-aminosulfonyl-2'-thiénylméthylamino, 2-furylméthylamino, 3'-pyridylméthylamino ou 4'-hydroxy-3'-pyridylamino et A est défini comme dans la revendication 1, et leurs sels avec des bases minérales ou organiques.

3. 6-alpha-méthoxy-pénicillines de formule générale I ou respectivement I' selon la revendication 1 et 2, caractérisées en ce qu'elles se présentent dans la configuration D = R.

4. L'acide 6β-{D-α-[3-(4-hydroxy-2-p-(4',5',6',-7'-tétrahydro-1,3-diazépine-2'-yl)-aminosulfonylanilino-5-pyrimidinyl)-uréido]-p-hydroxy-benzylamido}-6α-méthoxy-pénicillanique et son sel de sodium.

5. L'acide 6β-{D-α-[3-(4-hydroxy-2-p-sulfamoylanilino-5-pyrimidinyl)-uréido]-p-hydroxybenzylamido}-6α-méthoxy-pénicillanique et son sel de sodium.

6. Médicament caractérisé par une teneur en l'une ou plusieurs substances actives selon les revendications 1 à 5 conjointement aux excipients et/ou adjuvants usuels.

7. Procédé pour la préparation de composés selon la revendication 1, caractérisé en ce que:
on fait réagir un composé de formule générale II:

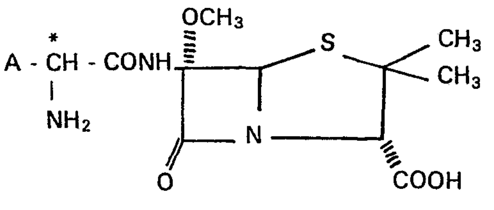
, (II)

dans laquelle A est défini comme dans la revendication 1, avec un dérivé de pyrimidine éventuellement préparé dans le mélange réactionnel, de formule générale III:

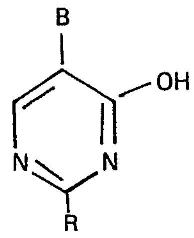
, (III)

ou

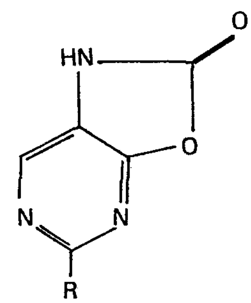
, (IIIa)

dans lesquelles:
R est défini comme dans la revendication 1 et
B représente le groupe -NCO ou un dérivé réactif du groupe NHCOOH, tel que par exemple les groupes -NHCOCl, -NHCOBr ou -NH-COO- 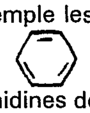 $-NO_2$, ou avec des mélanges de telles pyrimidines de formule générale III, dans laquelle B possède en partie l'une et en partie les autres des significations mentionnées plus haut, dans un solvant et dans un domaine de pH entre 2,0 et 9,0, à des températures entre −20°C et +50°C, et en ce qu'on transforme, si on le désire, un composé ainsi obtenu de formule générale I ou I' ensuite en des sels physiologiquement supportables avec des bases minérales ou organiques.

8. Procédé selon la revendication 7, caractérisé en ce qu'on fait réagir un composé de formule générale II ou l'un de ses sels avec des bases minérales ou organiques avec un composé de formule générale III,
a) dans l'eau ou dans des solvants miscibles à l'eau en présence d'eau dans un domaine de pH 6,5 à 8,0, ou
b) dans des solvants anhydres ou
c) dans un mélange d'eau et de solvants non miscibles à l'eau dans un domaine de pH entre 6,5 et 8,0, ou
en ce qu'on fait réagir un composé de formule générale II dans laquelle l'atome d'hydrogène du groupe carboxyle est remplacé par un groupe silyle ou par un autre groupe protecteur facilement clivable, avec un composé de formule générale III dans un solvant anhydre et ne contenant pas de groupe hydroxyle ou respectivement aprotique, éventuellement en présence d'une base.

**Revendications pour l'Etat contractant:** AT

1. Procédé pour la préparation de 6-alpha-méthoxy-pénicillines de formule générale :

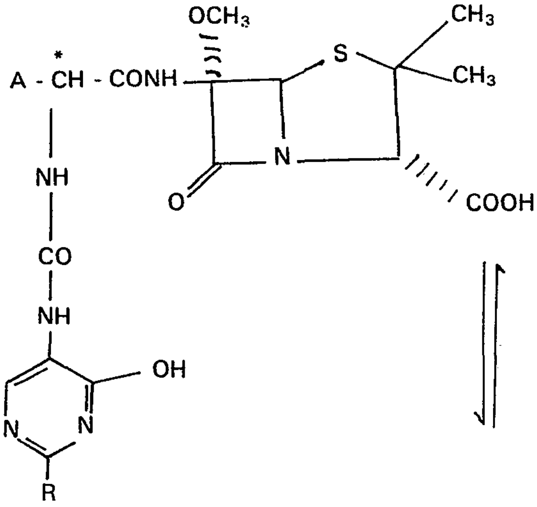
,(I)

ou

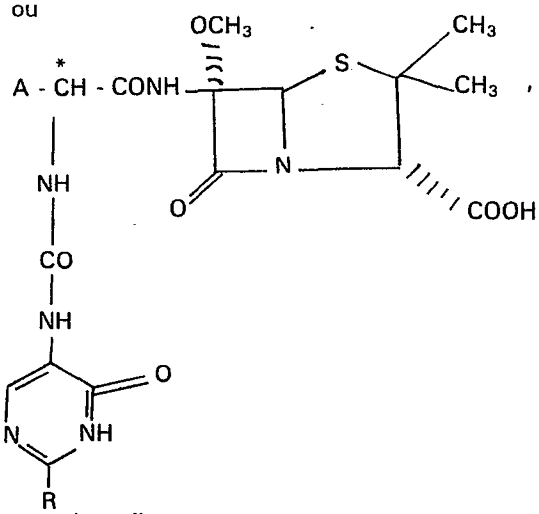
, (I')

dans laquelle:

A représente le groupe phényle, p-hydroxyphényle, 2- ou 3-thiényle,

R représente le groupe cyclopropyle, un groupe de formule générale

- NH - $R_1$, où:

$R_1$ représente un radical hydrocarboné avec 1 à 4 atomes de carbone, aliphatique, ramifié ou non ramifié, éventuellement substitué à partir de l'atome de carbone 2 par un groupe hydroxy, ou représente un radical cycloalcoyle avec 3 à 6 atomes de carbone éventuellement substitué par un groupe hydroxy, ou représente un groupe 3-pyridyle, 2-furylméthyle, 2-thiénylméthyle, 3-imidazolylméthyle, 2-thiazolylméthyle ou 3-pyridylméthyle éventuellement substitué par un groupe hydroxy, méthylsulfinyle, méthylsulfonyle ou aminosulfonyle,

ou R représente un groupe de formule générale:

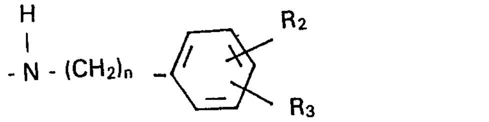
, où

n représente le chiffre 0 ou 1,

$R_2$ et $R_3$ qui peuvent être identiques ou différents représentent des groupes hydroxy, acétylamino, aminocarbonylamino, nitro, aminocarbonyle, cyano, méthylsulfinyle, méthylsulfonyle, aminosulfonyle, méthylaminosulfonyle, aminocarbonylméthylèneaminosulfonyle, 2'-hydroxyéthylaminosulfonyle, cyanaminosulfonyle, aminocarbonylaminosulfonyle, acétylaminosulfonyle, méthylsulfonylaminosulfonyle ou acétylhydrazinosulfonyle,

ou R représente un groupe de formule générale:

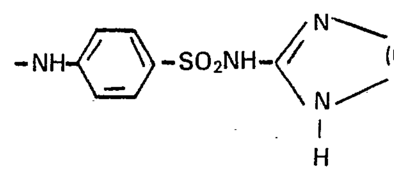
, où

m représente le nombre 2, 3 ou 4, ou

R représente un groupe de formule :

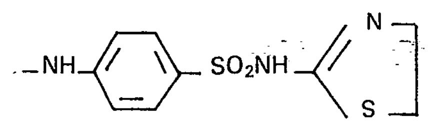

et de leurs sels avec des bases minérales ou organiques, caractérisé en ce que:

on fait réagir un composé de formule générale II:

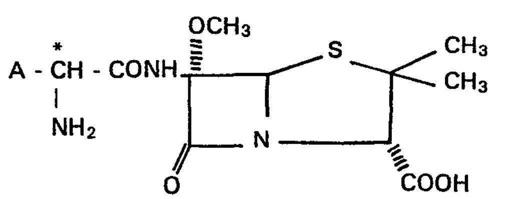
, (II)

dans laquelle A est défini comme plus haut, avec un dérivé de pyrimidine éventuellement préparé dans le mélange réactionnel, de formule générale III:

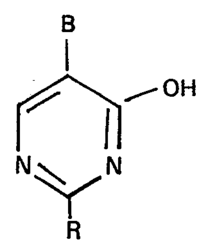
, (III)

ou

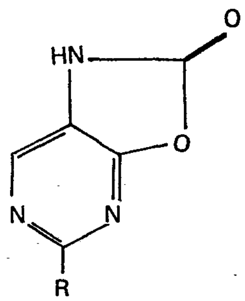

, (IIIa)

dans lesquelles:

R est défini comme plus haut et

B représente le groupe -NCO ou un dérivé réactif du groupe NHCOOH, tel que par exemple les groupes -NHCOCl, -NHCOBr ou -NH-COO- 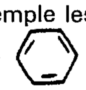 $-NO_2$, ou avec des mélanges de telles pyrimidines de formule générale III, dans laquelle B possède en partie l'une et en partie les autres des significations mentionnées plus haut, dans un solvant et dans un domaine de pH entre 2,0 et 9,0, à des températures entre —20°C et + 50°C, et en ce qu'on transforme, si on le désire, un composé ainsi obtenu de formule générale I ou I' ensuite en des sels physiologiquement supportables avec des bases minérales ou organiques.

2. Procédé selon la revendication 1, caractérisé en ce qu'on fait réagir un composé de formule générale II ou l'un de ses sels avec des bases minérales ou organiques avec un composé de formule générale III,

a) dans l'eau ou dans des solvants miscibles à l'eau en présence d'eau dans un domaine de pH 6,5 à 8,0, ou

b) dans des solvants anhydres ou

c) dans un mélange d'eau et de solvants non miscibles à l'eau dans un domaine de pH entre 6,5 et 8,0, ou

en ce qu'on fait réagir un composé de formule générale II dans laquelle l'atome de carbone du groupe carboxyle est remplacé par un groupe silyle ou par un autre groupe protecteur facilement clivable, avec un composé de formule générale III dans un solvant anhydre et ne contenant pas de groupe hydroxyle ou respectivement aprotique, éventuellement en présence d'une base.